(19) Europäisches Patentamt
European Patent Office
Office européen des brevets

(11) Veröffentlichungsnummer : **0 070 425**
**B1**

(12) **EUROPÄISCHE PATENTSCHRIFT**

(45) Veröffentlichungstag der Patentschrift : **13.03.85**

(51) Int. Cl.[4] : **C 07 C102/00**, C 07 C103/58, C 07 D295/18

(21) Anmeldenummer : **82105752.8**

(22) Anmeldetag : **29.06.82**

# (54) Verfahren zur Herstellung von N-substituierten Acryl- und Methacrylamiden.

(30) Priorität : **20.07.81 DE 3128574**

(43) Veröffentlichungstag der Anmeldung : **26.01.83 Patentblatt 83/04**

(45) Bekanntmachung des Hinweises auf die Patenterteilung : **13.03.85 Patentblatt 85/11**

(84) Benannte Vertragsstaaten : **BE DE FR GB NL**

(56) Entgegenhaltungen :
**EP-A- 0 018 629**
**GB-A- 2 021 105**
**US-A- 3 878 247**
**US-A- 4 143 220**

(73) Patentinhaber : **DEUTSCHE TEXACO AKTIENGESELLSCHAFT**
**Überseering 40**
**D-2000 Hamburg 60 (DE)**

(72) Erfinder : **Laping, Karlheinz, Dr.**
**Behringweg 1**
**D-4130 Moers 1 (DE)**
Erfinder : **Petersen, Olaf, Dr.**
**Comeniusstrasse 1**
**D-4050 Meerbusch 2 (DE)**
Erfinder : **Heinemann, Karl-Heinz, Dr.**
**Gartenstrasse 4**
**D-4133 Neukirchen-Vluyn (DE)**
Erfinder : **Humbert, Heiko, Dr.**
**Riepenhausenweg 5**
**D-2100 Hamburg 90 (DE)**
Erfinder : **Henn, Friedrich, Dr.**
**Erlenstrasse 10**
**D-4100 Duisburg 17 (DE)**

(74) Vertreter : **Schupfner, Gerhard et al**
**Patentanwälte Dipl.-Ing. Hans-Jürgen Müller**
**Dipl.-Chem.Dr.phil.nat. Gerhard Schupfner Dipl.-Ing.**
**Hans-Peter Gauger**
**Karlstrasse 5 D-2110 Buchholz in der Nordheide (DE)**

EP 0 070 425 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung bezieht sich auf ein Verfahren zur Herstellung von N-substituierten Acryl- und Methacrylamiden der allgemeinen Formel

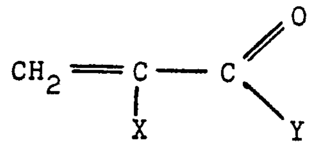

mit
X = Wasserstoff oder Methyl und
Y =

$NH-R-R_1$, 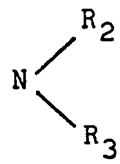

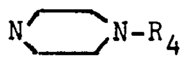 oder 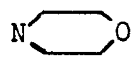

worin
R einen gerad-, verzweigtkettigen oder cyclischen Alkylenrest mit 2 bis 8 C-Atomen oder Phenylen,
$R_1$ Wasserstoff, eine Dialkylamino- oder Alkoxygruppe, wobei der Alkylrest jeweils 1 bis 4 C-Atome enthält,
$R_2$, $R_3$, $R_4$ jeweils einen Alkylrest mit 1 bis 4 C-Atomen, wobei $R_2$ und $R_3$ auch zusammen einen Ring mit 5 oder 6 C-Atomen bilden können,
bedeuten, wobei vorzugsweise Y

$NH-R-R_1$ oder 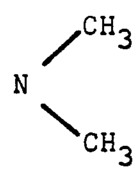

bedeutet, worin
R einen gerad- oder verzweigtkettigen Alkylenrest mit 2 bis 6 C-Atomen,
$R_1$ die Dimethylamino- oder die Methoxygruppe sind,
durch Herstellung von N,N'-disubstituiertem 3-Aminopropanamid und anschließende pyrolytische Zersetzung unter Aminabspaltung auf die in den Ansprüchen angegebene Weise.

Zur Herstellung von N-substituierten Acrylamiden ist eine Reihe von Verfahren bekannt. Der Stand der Technik wird z. B. ausführlich in der Europäischen Patentanmeldung 0 013 416 gewürdigt.

Eine große Zahl der Verfahren geht von Acrylsäureestern und Acrylsäureamiden aus. Dabei ist es im allgemeinen üblich, die Doppelbindung der eingesetzten Acryl- bzw. Methacrylsäurederivate zunächst durch Anlagerung von Wasser, Alkoholen oder Aminen zu schützen und die Schutzgruppe nach Herstellung des gewünschten Amids wieder abzuspalten.

Die von 3-hydroxy- oder 3-alkoxysubstituierten Carbonsäurederivaten ausgehenden Verfahren zur Herstellung von N-substituierten Acryl- bzw. Methacrylsäureamiden weisen gegenüber den Verfahren unter Einsatz von 3-Aminocarbonsäurederivaten erhebliche Nachteile auf.

So müssen die 3-Hydroxy- bzw. 3-Alkoxycarbonsäureamide, die in den Deutschen Offenlegungsschriften 2 819 735, 2 856 383, 2 911 642, 2 836 520, 2 918 486 und der Europäischen Anmeldung 0 013 416 beschrieben werden, in einem Mehrstufenprozeß erst hergestellt werden. Auch das in DE-OS 2 623 838 beschriebene Verfahren stellt in einer gesonderten Stufe zunächst 3-Alkoxypropansäureester her.

Diese Prozesse sind also schon hinsichtlich der Bereitstellung des erforderlichen Ausgangsmaterials sehr aufwendig. Weitere Nachteile ergeben sich bei der Reaktions- und Pyrolysestufe durch lange Reaktionszeiten, diskontinuierliche Reaktionsführung und die Abspaltung von Alkoholen bei der Pyrolyse, die vergleichbare Siedepunkte mit den eingesetzten Aminen haben oder mit diesen Azeotrope bilden, so daß ihre Abtrennung einen erhöhten Aufwand erfordert. Des weiteren ist zur Abspaltung von Wasser ein Katalysator notwendig, DE-OS 2 918 486. Ein weiterer Nachteil der von Estern ausgehenden Verfahren gemäß DE-AS 2 816 516 liegt im relativ niederen Siedepunkt der Ester begründet. Die Estermenge muß daher von vornherein so hoch gewählt werden, daß ein Esterüberschuß trotz der Destillationsverluste während der Dauer der Umsetzung aufrechterhalten werden kann, oder es muß mit dem Amin laufend Ester zugeführt werden. Unter totalem Rückfluß kann nicht gearbeitet werden, weil dann der abgespaltene Alkohol nicht aus dem Reaktionsgemisch entfernt werden kann. Schließlich muß das abdestillierte Ester/Alkohol-Gemisch aufgearbeitet werden.

Beim Verfahren gemäß US-PS 3 652 671, das von Methacrylsäure ausgeht, werden Methacrylamide nur in geringen Ausbeuten erhalten.

Gemäß US-PS 3 878 247 werden mindestens 2 Mol tertiäre Aminoalkylamine der allgemeinen Formel

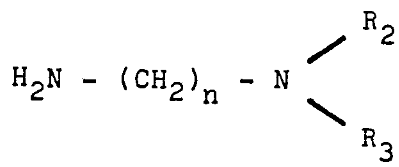

,

wobei $R_2$ und $R_3$ die oben angegebene Bedeutung haben und n 2 bis 6 ist, mit Acrylsäure, Methacrylsäure oder einem Ester dieser Säuren bei erhöhter Temperatur umgesetzt und die gebildeten N,N'-disubstituierten 3-Aminopropanamide bei 180 °C bis 300 °C unter Aminabspaltung pyrolytisch zersetzt.

Die Aufgabe der vorliegenden Erfindung bestand darin, ein Verfahren zu entwickeln, das die Nachteile des Standes der Technik nicht aufweist und die gewünschten N-substituierten Acryl- und Methacrylamide in hoher Ausbeute und Reinheit liefert.

In Lösung der gestellten Aufgabe betrifft die Erfindung ein Verfahren zur Herstellung der im Anspruch 1 definierten N-substituierten Acryl- und Methacrylamide, das dadurch gekennzeichnet ist, daß Acryl- bzw. Methacrylamid in kristalliner Form in Gegenwart von bekannten Amidierungskatalysatoren, insbesondere katalytisch wirksamen Protonen- bzw. Ammoniumionen-Spendern, oder in wäßriger Lösung mit mindestens 2 Mol eines Amins der allgemeinen Formel

$$YH$$

worin Y die oben angegebene Bedeutung besitzt, bei 100 bis 250 °C umgesetzt, Ammoniak und gegebenenfalls überschüssiges Amin und/oder Wasser destillativ entfernt, das erhaltene N,N'-disubstituierte 3-Aminopropanamid unter Aminabspaltung anschließend bei etwa 160 bis 350 °C zersetzt wird und die entstehenden Spaltprodukte über eine Kolonne durch fraktionierende Destillation aufgetrennt werden.

Es wurde somit überraschenderweise gefunden, daß man N-substituierte Acryl- bzw. Methacrylamide in technisch einfacher Weise und mit hohen Ausbeuten gewinnen kann, indem man kristallines Acryl- bzw. Methacrylamid mit Aminen in Gegenwart von Katalysatoren umamidiert und aus den intermediär erhaltenen N,N'-disubstituierten Aminopropanamiden zur Rückbildung der Doppelbindung pyrolytisch Amin abspaltet.

Unerwarteterweise wurde auch gefunden, daß die Umamidierung auch in wäßriger Lösung erheblich beschleunigt wird, so daß hierfür die Gegenwart zusätzlicher Katalysatoren nicht erforderlich ist. Es lassen sich also auch wäßrige Acryl- bzw. Methacrylamid-Lösungen verwenden, wie sie bei der in der Technik üblichen Herstellung von Acrylamid bzw. Methacrylamid aus dem entsprechenden Nitril normalerweise in 30 bis 50 % wäßriger Lösung anfallen. Damit entfällt die Notwendigkeit der Konzentrierung dieser Lösungen und die Kristallisierung des Amids. Für die Durchführung des erfindungsgemäßen Verfahrens in wäßriger Lösung sind die handelsüblichen 30 bis 50 % wäßrige Lösungen besonders geeignet. Es können jedoch auch entsprechend der Löslichkeit der eingesetzten Acryl- bzw. Methacrylamids Lösungen höherer Konzentrationen verwendet werden. Geringere als die handelsüblichen Konzentrationen sind möglich, jedoch in der Regel aus praktischen Gründen nicht sinnvoll, da ja das Wasser nach Durchführung der Reaktion wieder entfernt werden muß.

Ein weiterer Vorteil beim Einsatz der Acryl- und Methacrylamide liegt darin, daß diese meist keine destillierbaren Inhibitoren enthalten.

Es können primäre und sekundäre aliphatische Amine mit linearem, verzweigtem und cyclischem Kohlenstoffgerüst mit 1 bis 8 Kohlenstoffatomen verwendet werden. Das Kohlenstoffgerüst kann auch weitere funktionelle Gruppen, so z. B. Dialkylamino- oder Alkoxygruppen tragen. Als Beispiele können somit genannt werden :

Primäre Amine : Methylamin, Ethylamin, n-Butylamin, 2-Ethylhexylamin, Cyclohexylamin und insbesondere N,N-Dimethylpropandiamin, N,N,2,2-Tetramethylpropandiamin, Methoxypropylamin, N,N-Dimethyläthandiamin ;

Sekundäre Amine : Dimethylamin, Di-butylamin, Morpholin, N-Methylpiperazin ;

Aromatische Amine : Anilin, Toluidine, p-Methoxyanilin, p-Dimethylaminoanilin.

Man verwendet das Amin in stöchiometrischer Menge oder auch im Überschuß, also in 2- bis etwa 5-facher, insbesondere bis 4-facher molarer Menge, bezogen auf das Acryl- bzw. Methacrylamid.

Die Umsetzung zu den N,N'-disubstituierten 3-Aminopropanamiden wird bei Temperaturen zwischen 100 und 250 °C, vorzugsweise zwischen 100 und 190 °C, sowohl diskontinuierlich als auch kontinuierlich durchgeführt. Geeignete Reaktionsgefäße sind also Rührwerke, Autoklaven oder beheizbare Reaktionsrohre.

Die Umsetzung von kristallinem Acryl- bzw. Methacrylamid erfolgt in Gegenwart von sauren Katalysatoren. Die Umamidierung wird insbesondere durch Anwesenheit von Protonen bzw. Ammoniumionen beschleunigt. Es haben sich übliche Amidierungskatalysatoren als geeignet erwiesen. So verläuft die Umamidierung in Gegenwart von 1 bis 4 % Ammoniumverbindungen bis zu zwanzigmal schneller. Man kann insbesondere Aminhydrochlorid, Ammoniumchlorid oder Acryl- bzw. Methacrylsäure als Katalysatoren verwenden. Grundsätzlich als Katalysatoren geeignet sind organische und anorganische Protonsäuren (Brönstedtsäuren), wie Essigsäure sowie Ameisensäure, Propionsäure, Buttersäure usw. und Phosphorsäuren, wie ortho-Phosphorsäure, Polyphosphorsäuren, sowie auch Schwefelsäure und Salzsäure. Der Vorteil bei der Verwendung von Acryl- bzw. Methacrylsäure als Katalysator liegt darin, daß es sich ebenfalls zu dem gewünschten Produkt umsetzt und auf diese Weise keine Verunreinigungen entstehen. Auch Acryl- und Methacrylsäuresalze der jeweils eingesetzten Aminkomponente können als Katalysatoren zur Anwendung kommen. Weitere geeignete Katalysatoren sind Lewis-Säuren, wie Dialkylzinnoxide, insbesondere Dibutylzinnoxid, wenn auch — da es sich hier um eine homogene Katalyse handelt — die Abtrennung und Wiederverwendung einige Schwierigkeiten bereitet.

Die verwendeten Katalysatormengen liegen insbesondere bei etwa 1 bis etwa 5 Mol%, bezogen auf Acryl- bzw. Methacrylamid.

Die Umsätze zu den disubstituierten Propanamiden liegen zwischen 90 und 98 %.

Bei Verwendung von Wasser wird das überschüssige Amin und das Wasser abdestilliert. Wird kein Wasser verwendet, so muß Amin nicht unbedingt abdestilliert werden.

Das erhaltene N,N'-Disubstituierte 3-aminopropanamid wird pyrolytisch zersetzt, wobei unter Aminabspaltung die N-substituierten $\alpha,\beta$-ungesättigten Carbonsäureamide entstehen. Die Pyrolyse wird vorteilhaft kontinuierlich und insbesondere im Vakuum durchgeführt, und zwar wird bevorzugt das hergestellte disubstituierte Propanamid in einen erhitzten Kolben in dem Maße eingetropft, wie die Zersetzungsprodukte abgezogen werden. Die N-substituierten Acryl- bzw. Methacrylamide werden seitlich, die abgespalteten Amine über Kopf abgezogen. Man erhält auf diese Weise N-substituierte Acrylamide mit 95 bis 98 %iger Reinheit.

Diese Pyrolyse- und Trenntechnik wird insbesondere dann verwendet, wenn die N-substituierten Acrylamide mit dem jeweiligen Amin bei niederer Temperatur leicht rekombinieren können.

Ist keine schnelle Rekombination zwischen N-substituiertem Acryl- bzw. Methacrylamidderivat und Amin zu befürchten, können Pyrolyse und Fraktionierung in zwei aufeinanderfolgenden Verfahrensschritten durchgeführt werden. Die Pyrolyseprodukte werden in einer gemeinsamen Vorlage gesammelt und anschließend rektifiziert.

Die Pyrolyse erfolgt bei 160 bis 350 °C, vorzugsweise 160-270 °C, insbesondere bei etwa 200 bis 270 °C.

Das abgespaltene Amin und nicht pyrolysiertes bzw. durch Rekombination entstandenes Propanamid können ohne weiteres wieder in den Prozeß zurückgeführt werden.

Zur Verhinderung von Polymerisation während der Umsetzung können dem Reaktionsgemisch jeweils Inhibitoren wie z. B. Di-tert.-butylkresol, N,N-Diphenyl-phenylendiamin, Hydrochinonmonomethyläther zugefügt werden. Nach dem Verfahren der Erfindung ist jedoch der Zusatz von Inhibitoren nicht erforderlich. Da Inhibitoren oder deren Reaktionsprodukte gelegentlich die nachfolgende Polymerisation stören, ist dies ein besonderer Vorteil der Erfindung.

Beim erfindungsgemäßen Verfahren liegen die Ausbeuten hoch. Es werden Ausbeuten bis 95 % und mehr erzielt.

Die folgenden Beispiele erläutern die Erfindung.

## Beispiel 1

1 560 g (12 Mol) N,N,2,2-Tetramethylpropandiamin, 284 g (4 Mol) Acrylamid und 7 g Acrylsäure wurden im Verlauf von vier Stunden auf 150 bis 180 °C (bis zum Ende der Ammoniakentwicklung) erhitzt. Das Reaktionsprodukt, das überschüssiges Amin enthält, wurde zur Pyrolyse in gleichem Maße in einen auf 240 °C erhitzten Kolben getropft, wie über eine Kolonne bei 50 mm Druck seitlich N(N',N',2,2-Tetramethyl-3-aminopropyl)acrylamid und über Kopf des abgespaltenen Amins abgezogen wurden. Die Amidfraktion enthielt 95 % N(N',N',2,2-Tetramethyl-3-aminopropyl)acrylamid, was einer Ausbeute von 92 % entspricht.

Beispiel 2

1 560 g (12 Mol) N,N,2,2-Tetramethylpropandiamin, 284 g (4 Mol) Acrylamid und 1 g Ammoniumchlorid wurden im Verlauf von vier Stunden auf 150 bis 180 °C, bis zum Ende der Ammoniakentwicklung, erhitzt. Der Rückstand wurde wie in Beispiel 1 aufgearbeitet, wobei auch die gleichen Ausbeuten erreicht wurden.

Beispiel 3

390 g (3 Mol) N,N,2,2-Tetramethylpropandiamin, 71 g (1 Mol) Acrylamid und 1 g Dibutylzinnoxid wurden acht Stunden auf 150 bis 170 °C, dem Ende der Ammoniakentwicklung, erhitzt. Dann wurde der Rückstand wie in Beispiel 1 weiterverarbeitet. Die Amidfraktion, enthielt 95 % N(N',N',2,2-Tetramethyl-3-aminopropyl)acrylamid, was einer Ausbeute von 70 % entspricht.

Beispiel 4 (Vergleich)

1 560 g (12 Mol) N,N,2,2-Tetramethylpropandiamin und 284 g (4 Mol) Acrylamid wurden zum Rückfluß (ca. 145 °C) erhitzt. Die gaschromatographische Verfolgung der Reaktion zeigte, daß erst nach drei Tagen ca. 80 % umgesetzt waren. Die Reaktion war von einer viel stärkeren Bildung von Nebenprodukten als bei den Beispielen 1 und 2 begleitet.

Beispiel 5

1 560 g (12 Mol) N,N,2,2-Tetramethylpropandiamin und 947 g 30 %ige wäßrige Acrylamidlösung wurden in einem 5 l-Autoklaven unter autogenem Druck vier Stunden auf 160 °C erhitzt. Nach destillativer Entfernung des entstandenen Ammoniaks, des überschüssigen Amins und Wassers wurde ein öliger Rückstand erhalten, der in gleichem Maße in einen auf 230 °C erhitzten Kolben eingetropft wurde, wie über eine angeschlossene Kolonne bei 50 mm Druck seitlich das N(N',N',2,2-Tetramethyl-3-aminopropyl)acrylamid und über Kopf das abgespaltene Amin abgezogen wurde.

Es wurde N(N',N',2,2-Tetramethyl-3-aminopropyl)acrylamid mit einer Reinheit von 95 % erhalten. Die Gesamtausbeute betrug 88 %.

Beispiel 6

Ein Gemisch aus 390 Teilen (3 Mol) N,N,2,2-Tetramethylpropandiamin und 236,7 Teilen (1 Mol) 30 %ige wäßrige Acrylamidlösung wurde durch ein auf 180 °C erhitztes Rohr geleitet, wobei der Druck 10 bar und die Verweilzeit zwei Stunden betrug. Nach Austritt aus dem Reaktor wurden der entstandene Ammoniak, das überschüssige Amin und das Wasser destillativ entfernt. Der Rückstand wurde in gleichem Maße in einen auf 240 °C erhitzten Kolben getropft, wie über eine angeschlossene Kolonne bei 50 mm Druck seitlich N(N',N',2,2-Tetramethyl-3-aminopropyl)acrylamid und über Kopf das abgespaltene Amin abgezogen wurden. Es wurde N(N',N',2,2-Tetramethyl-3-aminopropyl)acrylamid in einer Reinheit von 98 % und einer Ausbeute von 91 % erhalten. Das überschüssige N,N,2,2-Tetramethylpropandiamin wurde nach Wasserabtrennung durch Azeotropdestillation mit Toluol, das Spaltamin direkt in den Rohrreaktor zurückgeführt.

Beispiel 7

1 224 g (12 Mol) N,N-Dimethylpropandiamin, 284 g (4 Mol) Acrylamid und 7 g Acrylsäure wurden vier Stunden zum Rückfluß erhitzt. Das Reaktionsprodukt wurde in gleichem Maße in einen auf 240 °C erhitzten Kolben getropft, wie über eine Kolonne bei 50 mm Druck seitlich N(N',N'-Dimethyl-3-aminopropyl)-acrylamid und über Kopf das abgespaltene Amin abgezogen wurden. Die Amidfraktion, 585 g, enthielt 95 % N(N',N'-Dimethyl-3-aminopropyl)-acrylamid, was einer Ausbeute von 89,1 % entspricht.

Beispiel 8

1 224 g (12 Mol) N,N-Dimethylpropandiamin, 340 g Methacrylamid wurden neun Stunden zum Rückfluß erhitzt. Zu Beginn wurden 3 g und im Verlaufe der Reaktion noch zweimal je 3 g Methacrylsäure zugegeben. Das Reaktionsprodukt wurde in gleichem Maße in einen auf 240 °C erhitzten Kolben getropft, wie über eine Kolonne bei 70 mm Druck seitlich N(N',N'-Dimethyl-3-aminopropyl)methacrylamid und über Kopf das abgespaltene Amin abgezogen wurden. Die Amidfraktion, 608 g, enthielt 96 % N(N',N'-Dimethyl-3-aminopropyl)methacrylamid. Dies entspricht einer Ausbeute von 85,8 %.

Beispiel 9

1 044 g (12 Mol) n-Butylamin, 284 g (4 Mol) Acrylamid und 5 g Acrylsäure wurden im Autoklaven vier Stunden auf 140 °C erhitzt. Das überschüssige Amin wurde abdestilliert und der Rückstand einer den vorhergehenden Beispielen analogen Pyrolysereaktion unterworfen. Dabei wurden bei einem Druck von 100 mm 1 523 g (90 %) N-n-Butylacrylamid erhalten.

Beispiel 10

710 g (10 Mol) Acrylamid und 5 g Dimethylaminhydrochlorid wurden in einen 5 l-Autoklaven gefüllt, 1 350 g (30 Mol) Dimethylamingas eingeleitet und vier Stunden auf 140 °C erhitzt. Anschließend wurde entspannt. Der Autoklaveninhalt wurde in einen auf 200 °C erhitzten Kolben getropft und gleichzeitig über eine angeschlossene Kolonne 85 g (85,9 %) N,N-Dimethylacrylamid abdestilliert. Das abgespaltene Dimethylamin wurde in einer Aceton/Trockeneis-Kühlfalle kondensiert.

Beispiel 11

710 g (10 Mol) Acrylamid und 3 375 g 40 %ige wäßrige Dimethylaminlösung wurden im Autoklaven vier Stunden auf 150 °C erhitzt. Das überschüssige Amin, das Wasser und der entstandene Ammoniak wurden abdestilliert. Der Rückstand wurde wie in Beispiel 10 aufgearbeitet. Man erhielt 871 g (88 %) N-N-Dimethylacrylamid.

Beispiel 12

71 g (1 Mol) Acrylamid, 300 g N-Methylpiperazin und 1 g Acrylsäure wurden zum Rückfluß erhitzt. Im Verlauf von acht Stunden wurden noch zweimal je 1 g Acrylsäure hinzugefügt. Das überschüssige Amin wurde abdestilliert. Der feste Rückstand wurde auf 300 °C erhitzt. Bei 100 mm Druck wurde zunächst das abgespaltene Amin, dann N(N'-Methylpiperazyl)acrylamid und zwar insgesamt 123 g (79,8 %) destilliert.

Beispiel 13 (Vergleich)

71 g (1 Mol) Acrylamid und 300 g N-Methylpiperazin wurden im Autoklaven auf 180 °C erhitzt. Die Umamidierung wurde gaschromatographisch verfolgt. Nach 48 Stunden hatten sich ca. 50 % umgesetzt. Auf eine Aufarbeitung wurde deshalb verzichtet.

**Ansprüche**

1. Verfahren zur Herstellung von N-substituierten Acryl- und Methacrylamiden der allgemeinen Formel

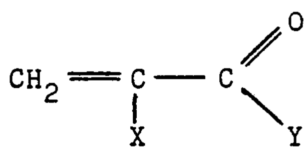

mit

X = Wasserstoff oder Methyl und

Y =

$NH-R-R_1$, 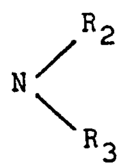 ,

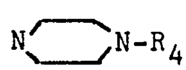 oder 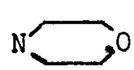

worin

R einen gerad-, verzweigtkettigen oder cyclischen Alkylenrest mit 2 bis 8 C-Atomen oder Phenylen,

$R_1$ Wasserstoff, eine Dialkylamino- oder Alkoxygruppe, wobei der Alkylrest jeweils 1 bis 4 C-Atome

enthält,

$R_2$, $R_3$, $R_4$ jeweils einen Alkylrest mit 1 bis 4 C-Atomen, wobei $R_2$ und $R_3$ auch zusammen einen Ring mit 5 oder 6 C-Atomen bilden können,

bedeuten, durch Herstellung von N,N'-disubstituiertem 3-Aminopropanamid und anschließende pyrolytische Zersetzung unter Aminabspaltung, dadurch gekennzeichnet, daß Acryl- bzw. Methacrylamid in kristalliner Form in Gegenwart von bekannten Amidierungskatalysatoren, insbesondere katalytisch wirksamen Protonen- bzw. Ammoniumionen-Spendern, oder in wäßriger Lösung mit mindestens 2 Mol eines Amins der allgemeinen Formel

YH

worin Y die oben angegebene Bedeutung besitzt, bei 100 bis 250 °C umgesetzt, Ammoniak und gegebenenfalls überschüssiges Amin und/oder Wasser destillativ entfernt, das erhaltene, N,N'-disubstituierte 3-Aminopropanamid unter Aminabspaltung anschließend bei 160 bis 350 °C zersetzt wird und die entstehenden Spaltprodukte über eine Kolonne durch fraktionierende Destillation aufgetrennt werden.

2. Verfahren nach Anspruch 1, dadurch gekennzeichnet, daß die Zersetzung im Vakuum erfolgt.

3. Verfahren nach Anspruch 1 oder 2, dadurch gekennzeichnet, daß die entstehenden Spaltprodukte direkt mit einer mit dem Pyrolysekolben unmittelbar verbundenen Kolonne sofort nach dem Entstehen aufgetrennt werden.

4. Verfahren nach einem der vorhergehenden Ansprüche, dadurch gekennzeichnet, daß die Umamidierung bei 100 bis 190 °C erfolgt.

5. Verfahren nach einem der vorhergehenden Ansprüche, dadurch gekennzeichnet, daß die Zersetzung bei 160 bis 270 °C erfolgt.

6. Verfahren nach einem der vorhergehenden Ansprüche, dadurch gekennzeichnet, daß es bei Einsatz von wäßrigem Acryl- oder Methacrylamid im Autoklaven bei autogenem Druck durchgeführt wird.

7. Verfahren nach einem der vorhergehenden Ansprüche, dadurch gekennzeichnet, daß Acryl- bzw. Methacrylamid und Amin im Molverhältnis 1 : 2 bis 1 : 4 eingesetzt werden.

8. Verfahren nach einem der vorhergehenden Ansprüche, dadurch gekennzeichnet, daß als Katalysatoren Ammoniumchlorid oder Hydrochloride der jeweils eingesetzten Aminkomponente zur Anwendung kommen.

9. Verfahren nach einem der Ansprüche 1 bis 7, dadurch gekennzeichnet, daß als Katalysatoren Acryl- bzw. Methacrylsäure oder Acryl- bzw. Methacrylsäuresalze der jeweils eingesetzten Aminkomponente zur Anwendung kommen.

10. Verfahren nach einem der vorhergehenden Ansprüche, dadurch gekennzeichnet, daß die Herstellung kontinuierlich erfolgt.

## Claims

1. Process for producing N-substituted acryl- and methacrylamides of the general formula

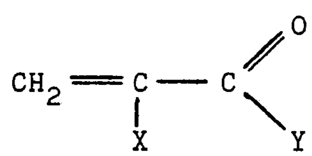

wherein

X is hydrogen or methyl and

Y is

$NH-R-R_1$, 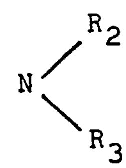 ,

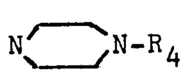 or 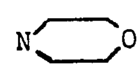 ,

wherein

R is a linear, branched, or cyclic alkylene residue with 2 to 8 carbon atoms, or phenylene,

$R_1$ is hydrogen, a dialkylamino- or alkoxy group, the alkyl residue having 1 to 4 carbon atoms each,

$R_2$, $R_3$, $R_4$ each are an alkyl residue with 1 to 4 carbon atoms, where $R_2$ and $R_3$ may also form together a ring with 5 or 6 carbon atoms,
by preparation of N,N'-disubstituted 3-aminopropanamide and subsequent pyrolytic decomposition while amine is split off, characterized by reacting acryl- or methacrylamide in crystalline form in the presence of known amidation catalysts, particularly catalytic protone or ammonium ion donators, or as an aqueous solution at 100-250 °C with at least 2 moles of an amine of the general formula

YH

wherein Y has the abovementioned significance, by distilling off amonia and, if necessary, excess amine and/or water, by subsequently decomposing the N,N'-disubstituted 3-aminopropanamide thus obtained at 160-350 °C, while amine is split off, and by fractionating the decomposition products thus obtained by distillation in a column.

2. Process according to claim 1, characterized by performing the decomposition under vacuum.

3. Process according to claim 1 or 2, characterized by directly fractionating the decomposition products as soon as they are formed in a column directly connected with the pyrolysis flask.

4. Process according to any one of the preceding claims, characterized by performing the transamidation at 100 to 190 °C.

5. Process according to any one of the preceding claims, characterized by performing the decomposition at 160 to 270 °C.

6. Process according to any one of the preceding claims, characterized by performing it in an autoclave under autogenous pressure when using aqueous acryl- or methacrylamide.

7. Process according to any one of the preceding claims, characterized by using acryl- or methacrylamide and amine at a mole ratio of 1 : 2 to 1 : 4.

8. Process according to any one of the preceding claims, characterized by using ammonium chloride or hydrochlorides of the respective amine component as catalysts.

9. Process according to any one of the claims 1 through 7, characterized by using acrylic or methacrylic acid or acrylic or methacrylic acid salts of the respective amine component as catalysts.

10. Process according to any one of the preceding claims, characterized by performing the preparation continuously.

**Revendications**

1. L'invention concerne un procédé pour préparer des acryl- et des méthacrylamides N-substitués de la formule générale

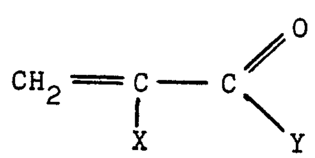

dans laquelle
X représente de l'hydrogène ou du méthyle, et
Y représente

NH-R-$R_1$, 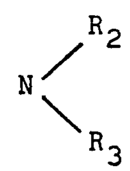 ,

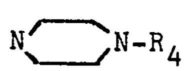 ou 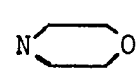 ,

dans laquelle
R représente un résidu d'alkylène rectiligne, ramifié ou cyclique ayant 2 à 8 atomes de carbone, ou du phénylène,
$R_1$ représente de l'hydrogène, un groupe de dialkylamine ou d'alkoxy, le résidu d'alkyl ayant 1 à 4 atomes de carbone chacun,
$R_2$, $R_3$, $R_4$ représentent un résidu d'alkyl chacun ayant 1 à 4 atomes de carbone, $R_2$ et $R_3$ pouvant également former ensemble un cycle ayant 5 ou 6 atomes de carbone,
par préparation de 3-aminopropanamide N,N'-disubstitué et par décomposition pyrolytique subséquente

au cours de laquelle l'amine est séparée, caractérisé en ce qu'on fait réagir à une température comprise entre 100 et 250 °C de l'acrylamide ou du méthacrylamide sous forme cristalline en présence des catalyseurs d'amidation connus, particulièrement des donneurs de protons ou d'ions d'ammonium catalytiques, ou en solution aqueuse avec au moins 2 moles d'une amine de la formule générale

YH

dans laquelle Y a la signification susmentionnée, qu'on sépare par distillation l'ammoniac et, le cas échéant, l'excès d'amine et/ou d'eau, qu'on décompose ensuite à une température comprise entre environ 160 et 350 °C le 3-aminopropanamide N,N'-disubstitué produit, ainsi donnant lieu à la séparation de l'amine, et qu'on fractionne les produits de décomposition ainsi obtenus par distillation dans une colonne.

2. Procédé selon la revendication 1, caractérisé en ce que la décomposition est effectuée sous vide.

3. Procédé selon la revendication 1 ou 2, caractérisé en ce que dès que les produits de décomposition soient formés ceux-ci sont décomposés dans une colonne directement raccordée au ballon de pyrolyse.

4. Procédé selon l'une quelconque des revendications précédentes, caractérisé en ce que la transamidation est effectuée à une température comprise entre 100 et 190 °C.

5. Procédé selon l'une quelconque des revendications précédentes, caractérisé en ce que la décomposition est effectuée à une température comprise entre 160 et 270 °C.

6. Procédé selon l'une quelconque des revendications précédentes, caractérisé en ce qu'on le réalise dans un autoclave sous pression autogène lorsqu'on utilise de l'acryl- ou du méthacrylamide aqueux.

7. Procédé selon l'une quelconque des revendications précédentes, caractérisé en ce qu'on utilise l'acryl- ou le méthacrylamide et l'amine à un rapport molaire de 1 : 2 jusqu'à 1 : 4.

8. Procédé selon l'une quelconque des revendications précédentes, caractérisé en ce qu'on utilise comme catalyseurs du chlorure d'ammonium ou des chlorhydrates de la composante d'amine employée.

9. Procédé selon l'une quelconque des revendications 1 à 7, caractérisé en ce qu'on utilise comme catalyseurs de l'acide acrylique ou méthacrylique ou des sels de l'acide acrylique ou méthacrylique de la composante d'amine employée.

10. Procédé selon l'une quelconque des revendications précédentes, caractérisé en ce que la préparation est effectuée en continu.